# FASCICULE DE BREVET EUROPEEN

(11) **EP 3 052 199 B1**
(45) Date de publication et mention de la délivrance du brevet: **13.12.2017**
(21) Numéro de dépôt: 14780462.9
(22) Date de dépôt: 01.10.2014
(51) Int. Cl.: A61Q 19/00, A61K 8/92, A61K 8/97, A61Q 19/08

(54) **UTILISATION D'UNE COMPOSITION HUILEUSE CONTENANT UN EXTRAIT D'HEMEROCALLE POUR AMELIORER LA FERMETE DE LA PEAU**
VERWENDUNG VON EINER ÖLIGEN ZUBEREITUNG ENTHALTEND EINEN HEMEROCALLIS-EXTRAKT ZUR VERBESSERUNG DER HAUTFESTIGKEIT
USE OF AN OILY COMPOSITION COMPRISING AN HEMEROCALLIS EXTRACT FOR IMPROVING FIRMNESS OF THE SKIN

(30) Priorité: 02.10.2013 FR 1359549
(43) Date de publication de la demande: 10.08.2016
(73) Titulaire: Société De Recherche Cosmétique S.à.r.L., 2314 Luxembourg (LU)
(72) Inventeur: LECONTE, Nadine, 92600 Asnieres Sur Seine (FR); ROSSIGNOL-CASTERA, Anne, 34160 Restinclieres (FR)
(74) Mandataire: Goulard, Sophie
(86) Numéro de dépôt international: PCT/EP2014/071009
(87) Numéro de publication internationale: WO 2015/049268

(56) Documents cités:
- FR-A1- 2 943 684
- JP-A- 2006 143 670
- JP-A- 2012 012 318
- DATABASE GNPD [Online] MINTEL; novembre 2011 (2011-11), Bloom: "Skin Ressetting Repairing", XP002722372, Database accession no. 1684089
- DATABASE GNPD [Online] MINTEL; novembre 2009 (2009-11), KAO: "Cream est Eternal Flow", XP002722293, Database accession no. 1211158
- DATABASE GNPD [Online] MINTEL; juillet 2013 (2013-07), Mantong, South Korea: "Transformation Cleansing Milk Oil", XP002722302, Database accession no. 2122962
- NAMSA N D ET AL: "An ethnobotanical study of traditional anti-inflammatory plants used by the Lohit community of Arunachal Pradesh, India", JOURNAL OF ETHNOPHARMACOLOGY, ELSEVIER IRELAND LTD, IE, vol. 125, no. 2, 7 septembre 2009 (2009-09-07), pages 234-245, XP026498478, ISSN: 0378-8741, DOI: 10.1016/J.JEP.2009.07.004 [extrait le 2009-07-14]

## Description

La présente invention se rapporte au domaine technique général des produits cosmétiques à usage topique, utilisables notamment sur la peau.

Plus précisément, la présente invention est relative à l'utilisation d'une composition huileuse à base d'un extrait lipophile d'hémérocalle à titre d'ingrédient actif pour la préparation d'une composition cosmétique à usage topique destinée à améliorer la fermeté et/ou l'élasticité de la peau, à un procédé cosmétique non thérapeutique pour améliorer la fermeté et/ou l'élasticité de la peau mettant en oeuvre une telle composition cosmétique, ainsi qu'à l'utilisation non thérapeutique d'une composition cosmétique à application topique comprenant un extrait lipophile d'hémérocalle pour améliorer la fermeté et/ou l'élasticité de la peau.

La peau est un véritable organe comprenant plusieurs couches intégrées, allant de la couche superficielle, l'épiderme, jusqu'aux couches plus profondes, le derme et l'hypoderme, et chacune de ces couches possède des propriétés spécifiques permettant à l'ensemble de réagir et de s'adapter aux conditions de son environnement.

L'hypoderme, qui est la couche la plus profonde de la peau, contient les adipocytes qui produisent des lipides pour que le tissu sous-cutané fabrique une couche grasse protégeant les muscles, les os et les organes internes contre les chocs.

L'épiderme est principalement composé de kératinocytes (90% des cellules épidermiques) qui synthétisent la kératine, de mélanocytes (2 à 3% des cellules épidermiques) responsables de la pigmentation de la peau, et des cellules de Langerhans qui jouent un rôle immunologique. L'épiderme, dont l'épaisseur est variable selon les différentes parties du corps, constitue la couche externe de la peau et par conséquent il joue un rôle fondamental pour assurer la protection et le maintien d'une bonne trophicité.

Le derme est un tissu conjonctif formant une couche plus épaisse que celle de l'épiderme, riche en nerfs, en vaisseaux sanguins et en glandes sudoripares. Il se compose de 2 régions :
- La zone superficielle : entre les crêtes épidermiques, ou « derme papillaire », formée de tissu conjonctif lâche. La zone superficielle renferme tout d'abord des fibres de collagène, fines, isolées et orientées mais aussi les anses de capillaires terminales et les terminaisons nerveuses ;
- La zone la plus profonde : ou " derme réticulaire" est formée d'un tissu conjonctif dense où les fibres de collagène plus épaisses en faisceaux et les fibres élastiques s'entrecroisent dans toutes les directions dans les plans grossièrement parallèles à la surface cutanée. Le derme réticulaire contient aussi de petites artérioles et veinules, des petits nerfs des follicules pilo-sébacés.

Le derme comprend :
- des macromolécules de type protéique, en particulier des fibres de collagène, de l'élastine et de la fibronectine conférant à la peau souplesse, élasticité et assise ;
- des mucopolysaccharides, sorte de gel dans lequel baignent les macromolécules - ce « gel » est formé de glycosaminoglycanes, protéines qui à la manière d'une éponge vont capter l'eau dans le derme et ainsi agir comme réservoir d'hydratation ;
- diverses cellules dont les fibroblastes qui participent à la synthèse des macromolécules et les cellules du système immunitaire (lymphocytes, mastocytes, macrophages tissulaires).

Le réseau élastique du derme comprend 3 grandes sortes de fibres : les fibres oxytalanes, les fibres d'élaunine et les fibres élastiques proprement dites, matures. Les fibres oxytalanes sont situées dans le derme papillaire. Elles forment de fines arborisations perpendiculaires à la jonction dermo-épidermique (JDE). Les fibres oxytalanes du derme papillaire sont exclusivement constituées de microfibrilles. Les fibres d'élaunine sont des fibres élastiques immatures, plus courtes et moins larges que les fibres élastiques matures et avec des plages amorphes moins développées que les zones fibrillaires. Enfin, Les « fibres élastiques » sont situées au niveau du derme réticulaire, des septas interlobulaires de l'hypoderme, avec un renforcement autour des follicules pileux, des glandes sébacées et des glandes sudorales. Elles sont principalement constituées d'élastine et se présentent comme des faisceaux ondulés situés entre les fibres de collagène.

Les fibres de collagène, qui représentent 70% du poids sec du derme, assurent la résistance mécanique et la texture de la peau, l'élastine est responsable de l'élasticité, les protéoglycanes jouent un rôle majeur de structure et d'hydratation de la peau et les glycoprotéines telles que la fibrilline et les filaments de vimentine, sont fortement impliquées dans le maintien de l'élasticité et dans l'organisation de la contraction et de la cohésion du derme.

Plus particulièrement, la fibrilline (ou fibrilline-1) est une glycoprotéine de 340 kDa. Les fibres d'oxytalane sont des fibres très riches en fibrilline-1. Ces fibres jouent un rôle crucial dans le maintien des fonctions du derme (fonction de soutien) et permettent au derme d'apporter à la peau fermeté, élasticité et souplesse. Cependant plusieurs facteurs, tels que la formation de méthylglyoxal, sont capables de dégrader ce réseau élastique. Son importance dans l'assemblage et la fonctionnalité du réseau élastique, font de la fibrilline-1 un bio-marqueur très utilisé en recherche dermo-cosmétique pour étudier l'état structural de la matrice extracellulaire du derme.

La vimentine est une protéine essentielle du cytosquelette du fibroblaste. Elle a un rôle capital dans l'organisation de la contraction et de la cohésion du derme, et elle organise les fibres de collagène. Elle permet au fibroblaste de conserver une bonne contractilité, ce qui est caractéristique d'un fibroblaste jeune. Elle a également un rôle important dans de nombreux métabolismes cellulaires (déplacement cellulaire, division, cicatrisation...). La vimentine fait partie des filaments intermédiaires qui constituent, avec les microtubules et les microfilaments d'actine, le cytosquelette. Le cytosquelette d'une cellule est l'ensemble organisé des polymères biologiques qui lui confèrent l'essentiel de ses propriétés mécaniques (contractilité, élasticité, maintien de sa forme et de sa structure interne...). La vimentine est liée au noyau, au réticulum endoplasmique ainsi qu'aux mitochondries. Elle joue donc un rôle important dans le soutien et l'ancrage de la position des organites au sein du cytoplasme. Cette molécule est responsable du maintien de la forme cellulaire et de l'intégrité du cytoplasme. Il a été montré que la vimentine est une cible majeure de produits finaux de la glycation : les AGE (*Advanced Glycation End products*). Les AGEs formés par le MG et accumulés sur la vimentine provoquent son agrégation, réduisent les capacités contractiles de la cellule et désorganisent le réseau des fibres de collagènes (*Kueper et al.,* 2007 - Publication consultable en annexe).

Le vieillissement de la peau peut être extrinsèque c'est-à-dire provoqué par l'environnement, y compris les agressions climatiques, qui peuvent notamment contribuer à accélérer la dégradation du collagène du derme, et en particulier l'exposition au soleil, les variations de températures mais également intrinsèque, notamment découler de la formation de méthylglyoxal qui est un sous-produit de différentes voies métaboliques telles que la glycolyse et la peroxydation des lipides et dont la présence entraine une dégradation du réseau élastique du derme (glycation des protéines).

Les produits d'origine naturelle ou contenant des composés naturels sont par ailleurs de plus en plus appréciés par les consommateurs et consommatrices de produits cosmétiques. C'est pourquoi on trouve sur le marché des produits cosmétiques de plus en plus de produits contenant des substances naturelles ou d'origine naturelle.

De telles compositions contiennent classiquement au moins une substance anti-oxydante, en particulier des molécules complexes d'origine végétale telles que par exemple les polyphénols, les flavonoïdes, les anthocyanosides, les caroténoïdes, etc... D'autres extraits végétaux tels que ceux de issus de l'arbre à soie (*Albizia julibrissin*), du café grâce à leur composition phénolique, ou encore la fraction éthanolique de mélisse ont une action sur la glycation des protéines.

Ces molécules ne sont toutefois pas toujours assez stables dans le temps et perdent ainsi progressivement leur efficacité.

Il existe donc un besoin pour des compositions cosmétiques contenant un extrait végétal d'origine naturelle ayant la capacité de diminuer au moins en partie et de façon stable dans le temps la glycation des protéines et d'inhiber, au moins en partie et de façon stable dans le temps, les effets néfastes de l'accumulation de méthylglyoxal dans les cellules de la peau, notamment la perte d'élasticité ou de fermeté de la peau.

Hémérocalle est le nom commun donné à la fleur du genre *Hemerocallis* qui appartient à la famille des liliacées. Elle est aussi appelée Lys d'un jour. En effet, elle doit son nom au grec 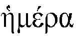 (Hemera) «jour» et καλóς (kalos) «beauté». On l'appelle lys d'un jour, car sa fleur est éphémère et ressemble au lys, mais le feuillage est totalement différent. La fleur de l'hémérocalle ne vit qu'une seule journée, mais de nouvelles fleurs se succèdent pendant plusieurs semaines. Les fleurs se fanent à la tombée du jour et laissent la place à d'autres dès le lendemain matin.

Les Hémérocalles sont connues et cultivées depuis des siècles. Originaires d'Asie, les premières espèces étaient déjà cultivées, décrites et peintes à l'époque de Confucius (551 - 479 Av JC).

*Hemerocallis fulva* est connue, notamment en médecine traditionnelle chinoise, pour ses effets calmants, cicatrisants et bactéricides. Elle est également utilisée dans le traitement de la dépression, de l'inflammation, de l'insomnie et des furoncles.

En particulier, la demande de brevet US2011/0076349 décrit une composition à administrer par voie orale contenant, à titre de principe actif, un extrait aqueux des parties aériennes *d'Hemerocallis fulva* et ayant des effets antidépresseurs ou défatiguants basés sur l'amélioration du sommeil.

Les études effectuées par la Demanderesse ont montré qu'un extrait huileux d'Hémérocalle, plus précisément d'*Hemerocallis fulva,* permet de pallier, au moins en partie, les effets néfastes de l'accumulation de méthylglyoxal dans le derme et ainsi i) de maintenir la qualité et la quantité de fibrilline-1 et donc la fonctionnalité de l'élastine et ii) de protéger les fibroblastes vis-à-vis de la glycation de la vimentine et ainsi participer au maintien de leurs fonctionnalités, en particulier de leurs propriétés contractiles.

La présente invention a donc pour premier objet l'utilisation, à titre d'ingrédient actif, d'une composition huileuse à base d'un extrait lipophile *d'Hemerocallis fulva* et d'un véhicule huileux comprenant au moins une huile végétale (huile vectrice), pour la préparation d'une composition cosmétique à usage topique destinée à améliorer la fermeté et/ou l'élasticité de la peau.

L'invention a également pour objet un procédé cosmétique non thérapeutique pour améliorer la fermeté et/ou l'élasticité de la peau, consistant à appliquer sur les zones de la peau concernées au moins une composition cosmétique topique renfermant une composition huileuse à base d'un extrait lipophile *d'Hemerocallis fulva* et d'un véhicule huileux comprenant au moins une huile végétale.

Enfin, l'invention a également pour objet l'utilisation non thérapeutique d'une composition cosmétique à application topique renfermant une composition huileuse à base d'un extrait lipophile *d'Hemerocallis fulva* et d'un véhicule huileux comprenant au moins une huile végétale, pour améliorer la fermeté et/ou l'élasticité de la peau.

Selon l'invention, la teneur en composition huileuse dans la composition cosmétique peut varier varie de 0,01 à 50% en masse, de préférence de 0,1 à 10 % en masse et encore plus préférentiellement de 0,5 à 4 % en masse par rapport à la masse totale de la composition cosmétique.

Les compositions cosmétiques à application topique comprenant une telle composition huileuse à titre d'ingrédient actif présentent d'excellentes propriétés anti-glycation utilisables en cosmétique pour améliorer la fermeté de la peau et/ou son élasticité, notamment en diminuant ou en inhibant les effets néfastes de la formation de méthylglyoxal dans sur certaines protéines structurelles du derme telles que la fibrilline-1 et la vimentine, ces propriétés étant tout particulièrement marquées dès que la quantité de composition huileuse est de l'ordre de 1 % en masse par rapport à la masse totale de la composition cosmétique. En effet, la composition huileuse conforme à l'invention est composée d'une combinaison d'antioxydants qui agissent de façon synergique contre les phénomènes oxydatifs auxquels les cellules cutanées sont exposées, en particulier contre la glycation des protéines. Elle apporte à la peau à la fois des antioxydants apolaires tels que la vitamine E et des caroténoïdes (lutéine, zéaxanthine), mais aussi des antioxydants plus polaires tels que les polyphénols dont les acides chlorogéniques en quantité importante. Cette combinaison d'agents antioxydants, véhiculée par la ou les huiles végétales du véhicule huileux, est particulièrement stable dans le temps et efficace pour améliorer la fermeté et/ou l'élasticité de la peau.

De plus, aux doses utilisées, les essais effectués ont montré que la composition huileuse utilisée dans la présente invention ne présente aucune cytotoxicité.

Les études effectuées par la demanderesse ont montré que l'on peut utiliser toutes les parties aériennes de la plante, aussi bien les tiges, les feuilles et les fleurs. L'espèce *Hemerocallis fulva* est facilement disponible et la conservation des plantes ne soulève généralement pas de difficulté technique.

La composition huileuse conforme à l'invention peut être préparée selon le procédé d'extraction décrit dans la demande internationale WO 2010/112760 consistant, dans une première étape, à mélanger et imprégner la plante, préalablement séchée et broyée, dans au moins une huile végétale naturelle à une température supérieure à son point de fusion, puis à chauffer le mélange à haute température pendant une durée très courte, et à former une microdispersion à une température supérieure à la température de fusion de l'huile végétale. Toutes ces étapes se font de préférence à l'abri de l'air, plus particulièrement à l'abri de l'oxygène pour éviter toute réaction d'oxydation de l'extrait. On peut par exemple travailler sous atmosphère d'azote, dans un réacteur clos avec extraction continue de l'oxygène par un flux d'azote, ou encore sous vide.

Les huiles végétales naturelles utilisables dans ce procédé sont de préférence choisies parmi l'huile d'avocat, l'huile de rosier muscat, l'huile de noisette, l'huile de colza, l'huile de macadamia, l'huile d'argan, l'huile de tournesol, l'huile de cameline, l'huile de bourrache, l'huile d'amande douce, l'huile de carthame, l'huile de calophyllum et leurs mélanges.

Selon, une forme de réalisation préférée de l'invention, l'huile végétale est choisie parmi l'huile d'avocat, l'huile de rosier muscat et leurs mélanges.

Selon une forme de réalisation tout particulièrement préférée de l'invention, le véhicule huileux est un mélange d'huile d'avocat et d'huile de rosier muscat.

Les huiles végétales utilisées selon l'invention, et en particulier les huiles d'avocat et de rosier muscat, sont des produits disponibles dans le commerce.

L'avocat est le fruit de l'avocatier (*Persea americana*), un arbre originaire d'Amérique du Nord (Mexique). Riche en acide oléique, en caroténoïdes et en éléments nutritifs, l'huile d'avocat est extraite de la pulpe du fruit (chair) car paradoxalement, le noyau ne contient que très peu de corps gras.

L'huile de rosier muscat est extraite des graines du cynorhodon (un « faux » fruit) du rosier muscat qui est un églantier sauvage. Elle est l'une des huiles végétales les plus riches en acides gras poly-insaturés essentiels (acides linoléique et linolénique) et contient des insaponifiables. Elle a des propriétés régénérantes.

L'utilisation de ces huiles végétales comme solvant de l'extrait de d'hémérocalle présente l'avantage d'être compatible avec les normes des produits certifiés Ecocert s'appliquant aux produits cosmétiques à la fois écologiques et biologiques.

La première étape du procédé ci-dessus, consistant à imprégner la plante, de préférence broyée, se déroule à température ambiante, ou à chaud, à une température supérieure à celle de fusion des huiles utilisées. On travaille de préférence à température ambiante, de l'ordre de 25°C, les huiles végétales, et en particulier les huiles d'avocat et de rosier muscat, étant liquides à cette température.

Selon une forme de réalisation préférée, le broyage de la plante est effectué à une température d'environ -80°C, jusqu'à obtention d'une poudre présentant une granulométrie moyenne inférieure à 100 µm environ.

La deuxième étape comporte un chauffage à une température élevée, par exemple de l'ordre de 140 à 170°C, pendant une courte durée, généralement inférieure à 5 minutes. Le temps de montée en température doit aussi être très court. La technique du chauffage par micro-ondes est bien adaptée à ces conditions. La technique des micro-ondes facilite aussi la troisième étape de microdispersion des particules dans la ou les huiles végétales utilisées, avec rupture des membranes cellulaires. Cette troisième étape peut aussi comprendre un traitement par cavitation ultrasonore, de préférence dans un réacteur fermé équipé d'un générateur d'ultrasons à basse fréquence. Cette troisième étape peut éventuellement être réalisée simultanément avec les deux autres, l'ensemble des étapes, ou chacune d'elles, pouvant être réalisé plusieurs fois si nécessaire.

A la fin de l'extraction, l'extrait lipophile est de préférence filtré sur un filtre ayant une porosité inférieure à environ 5 µm, de façon à obtenir un extrait lipophile limpide.

Ce procédé est particulièrement efficace sur une matière première telle que l'hémérocalle contenant entre 85 et 90 % d'eau avant broyage et séchage. Après séchage, avant l'étape d'imprégnation dans la ou les huiles végétales, la teneur en eau est ramenée en dessous de 12 %.

Lorsque l'extraction est réalisée en utilisant un mélange d'huile d'avocat et de rosier muscat, on obtient un extrait lipophile de couleur vert foncé ayant une densité relative à 20°C variant de 0,900 à 0,990 g/cm³ environ (NF ISO 6883), soluble dans les huiles et phases grasses, et ayant les caractéristiques suivantes :
- Teneur en eau et en volatils (NF EN ISO 662) : max. 0,20 % ± 0,04 % ;
- Acidité oléique (NF EN ISO 660) : max. 3,0% ± 0,6% ;
- Indice de peroxyde (NF EN ISO 3960) : max 10,0 ± 4,0 méqO₂/kg ;
- Teneur en phénols (dosages par HPLC) :
   * Catéchines : 150 mg/kg
   * Quercétine : 250 mg/kg
   * Rutine : 150 mg/kg
   * Acide chlorogénique : 416 mg/kg.
- Teneur totale en caroténoïdes (lutéine et zéaxanthine) : 5,1 mg/kg de composition huileuse (mesuré par HPLC) ;
- Teneur en β-carotène : 8,6 mg/kg de composition huileuse (mesuré par HPLC) ;
- Composition en acides gras de la composition huileuse :
   * acide palmitique 6-26 %
   * Acide palmitoléïque 1,5-10 %
   * Acide stéarique max. 2 %
   * Acide oléique 30-65 %
   * Acide linoléique 13-27 %
   * Acide α-linolénique 5-14 %
   * Acide arachidique max. 1 %
   * Acide érucique max. 1 %

De plus, la technique utilisée permet d'obtenir une composition huileuse bien plus concentrée en principes actifs qu'un simple macérât huileux. Ainsi, suivant l'invention on associe les avantages d'une huile végétale et de molécules hydrophiles (flavonoïdes, composés phénoliques). Enfin, le véhicule huileux évite de devoir ajouter un conservateur à la composition, celui-ci permettant en effet de stabiliser dans le temps les propriétés antioxydantes de l'extrait lipophile d'hémérocalle et donc l'efficacité des compositions cosmétiques la renfermant.

Selon une forme de réalisation préférée de l'invention, le véhicule huileux comprend un mélange d'huile d'avocat et d'huile de rosier muscat. Dans ce cas, le rapport massique huile d'avocat/huile de rosier muscat au sein de la composition huileuse varie de préférence de 4/1 à 1/1 environ, et encore plus préférentiellement de 2/1 à 1/1.

Au sein de la composition huileuse utilisable selon l'invention, la teneur en extrait lipophile d'hémérocalle (molécules lipophiles extraites de la plante hémérocalle, soit principalement des caroténoïdes et du β-carotène) peut varier de 5 à 20 ppm, et plus préférentiellement, de 15 à 20 ppm.

Les compositions utilisables selon l'invention peuvent contenir, outre la composition huileuse conforme à l'invention, un ou plusieurs actifs secondaires renforçant ou complétant avantageusement son activité, et compatibles, c'est-à-dire non susceptibles de réagir les uns avec les autres ou de masquer ou limiter ses effets.

Plus particulièrement, les actifs secondaires peuvent être choisis parmi un agent anti-âge, ou parmi l'acétyl tetrapeptide-5 (CAS N° 820959-17-9), un extrait de plancton, un extrait de maca qui a un effet sur la prévention de dérèglements liés à une déficience de la microcirculation cutanée et au stress oxydatif qui peut en résulter, un extrait de pétales de coquelicot agissant sur la nutrition cellulaire, une combinaison d'extraits d'anchuse, de coquelicot et de passiflore qui, en association, ont un effet dans la prévention des signes du vieillissement cutané, un extrait de graines de mimosa, un extrait de pétales de souci, un extrait de barbitamao, un extrait de kigelia africana, un extrait d'avoine, les cellules de cacao, la vitamine C, la vitamine E et l'hexylrésorcinol.

L'agent anti-âge peut être par exemple un lipide tel que le géranyl géranyl isopropanol (Juvinity®) qui réduit la formation des peroxydes intracellulaires et retarde ainsi le vieillissement cellulaire.

Les compositions cosmétiques utilisables selon la présente invention possèdent une excellente tolérance cutanée, notamment en raison du film lipidique résultant du support huileux (huiles végétales, en particulier huile d'avocat et huile de rosier muscat), elles ne présentent aucune phototoxicité et leur application sur la peau, pour des périodes de temps prolongées, n'implique aucun effet systémique.

Les compositions utilisables selon la présente invention peuvent se présenter sous les formes galéniques classiquement utilisées pour une application topique, c'est-à-dire sous forme de gel, d'émulsion (en particulier crème ou lait), d'émulsion biphasique huile-dans-eau ou eau-dans-huile, d'huile corporelle, de masque, de pommade, d'onguent, de lotion, de solution concentrée, de nanocapsules, de liposomes, lesdites formes galéniques contenant en outre des excipients et supports usuels et acceptables sur le plan cosmétologique. Elles sont utilisées de préférence sous forme de crèmes, de sérums, de lotion ou de gel.

Ces formes d'administration par voie topique sont préparées par les techniques usuelles, et par exemple, dans le cas d'une crème, par dispersion d'une phase grasse dans une phase aqueuse pour obtenir une émulsion huile-dans-eau, ou inversement pour préparer une émulsion eau-dans-huile. Dans le cas de crèmes, on peut utiliser des émulsions à structure lamellaire obtenues avec des lécithines hydrogénées ou des sucro-esters, contenant peu de produits éthoxylés ou n'en contenant pas du tout.

Les compositions cosmétiques utilisables suivant l'invention peuvent aussi prendre la forme de lotion ou solution dans laquelle les extraits sont sous forme encapsulée dans des microsphères. Les microsphères suivant l'invention peuvent par exemple être constituées de corps gras, d'agar et d'eau. La composition huileuse utilisable conformément à l'invention peut être incorporée dans des vecteurs de type liposomes, glycosphères, dans des chylomicrons, des macro-, micro-, nano-particules ainsi que les macro-, micro- et nanocapsules et aussi être adsorbés sur des polymères organiques poudreux, les talcs, bentonites et autres supports minéraux.

Ces émulsions jouissent d'une bonne stabilité et peuvent être conservées pendant le temps nécessaire pour l'utilisation à des températures comprises entre 0 et 50 °C sans qu'il y ait sédimentation des constituants ou séparation des phases.

Les compositions topiques utilisables selon l'invention peuvent comprendre des excipients appropriés pour une application topique externe, en particulier des excipients acceptables sur le plan cosmétologique. Ces excipients appropriés pour la formulation sont bien connus de l'homme du métier et comprennent en particulier des agents de pénétration tels que le phytantriol, l'octyl dodécanol et l'escine ; les épaississants tels que les gommes naturelles et les polymères de synthèse ; les tensioactifs ; les émollients tels que l'octanoate de cétéaryle, le myristate d'isopropyle, l'isononanoate de cétéaryle, la diméthicone, la cyclométhicone, le 3-diisostéarate de polyglycéryle, le polyisobutène hydrogéné, l'alcool cétylique, le palmitate cétylique, le phosphate cétylique ; les émulsionnants tels que des dérivés de polyglycérol ; les conservateurs tels que le phénoxyéthanol et l'acide déhydroacétique ; les colorants ; les parfums ; etc...

Comme autres ingrédients utilisables dans les compositions de l'invention, on peut citer les agents hydratants tels que la glycérine, le butylène glycol, le sel de sodium de l'acide pyrrolidone carboxylique (sodium PCA), ainsi que des associations de dérivés glucosiques à effet hydratant et restructurant comme le produit Aquaxyl® (xylitylglucoside et anhydroxylytol et xylitol), et également les vitamines antioxydantes telles que la vitamine E, par exemple l'acétate de tocophérol ou le tocotriénol, la vitamine C, les polyphénols naturels.

On peut aussi ajouter à la composition des glucides choisis principalement parmi le glucose, le glycogène et le tréhalose, ou encore un palmitoyl pentapeptide-3 tel que le Matrixyl® ou des dérivés tels que le palmitoyl GHK (possédant la chaîne Glycyl-Histidyl-Lysine) et le palmitoyl GQPR (Glycyl-Glutamyl-Prolyl-Arginine) ou le palmitoyl VGVAPG (Valyl-Glycyl-Valyl-Alanyl-Prolyl-Glycine) associé à un céramide-2, ainsi que d'une manière générale toute association avec un céramide.

On peut également ajouter à la composition des agents de protection contre les rayons ultraviolets, et par exemple des filtres solaires UV-A et UV-B hydrophiles ou lipophiles, ou des pigments formant écran anti-ultraviolet.

Les filtres solaires peuvent être choisis par exemple parmi la benzophénone ou un dérivé de benzophénone tel que la 2-hydroxy-4-méthoxy-benzophénone (Eusolex® 4360), ou un ester d'acide cinnamique et plus particulièrement le méthoxycinnamate d'octyle (Eusolex® 2292), le méthoxycinnamate d'éthyl-2-hexyle (Parsol MCX®), ou encore un cyano-β,β-diphénylacrylate tel que l'octocrylène (Eusolex® OCR), le 4-méthylbenzylidène camphre (Eusolex 6300®), et des dérivés du dibenzoylméthane tels que le 4-isopropyl dibenzoylméthane (Eusolex 8020), le t-butyl-méthoxy dibenzoylméthane (Parsol 1789®), et le 4-méthoxy-dibenzoylméthane.

Les pigments peuvent être choisis par exemple parmi le dioxyde de titane, l'oxyde de zinc et l'oxyde de zirconium.

L'utilisation cosmétique de la composition cosmétique conforme à l'invention comprend tous les soins de la peau, en particulier les soins du visage, y compris les produits solaires, protecteurs et bronzants, les produits anti-âge, anti-séborrhéiques, toniques, les produits assurant l'amélioration de l'aspect de la peau y compris le traitement acnéique et les rougeurs cutanées.

Les exemples suivants illustrent l'invention plus en détail sans en limiter la portée. Dans tous les exemples de compositions qui suivent, les parties sont exprimées en masse, sauf indication contraire.

### EXEMPLES

### EXEMPLE 1 : Préparation d'une composition huileuse comprenant un extrait lipophile d'hémérocalle

Une composition huileuse à base d'extrait lipophile d'*Hemerocallis fulva* a été préparée comme indiqué ci-après.

On a utilisé l'ensemble des parties aériennes de la plante entière, préalablement séchée, puis broyée à froid. La technique du séchage/broyage à froid est préférable car elle permet de préserver les anti-oxydants présents dans la plante. Suivant cette technique, les plantes sont soumises à un premier broyage, puis à une déshydratation à température ambiante à l'abri de la lumière, de manière à ramener la teneur en eau vers 10%, et broyées à froid, à une température de -80°C, au moyen d'un broyeur à couteaux.

La teneur en eau était inférieure à 12 % après broyage et séchage à froid, ce pourcentage pouvant notamment varier de 8,5 à 12 % environ.

20 kg d'hémérocalle ainsi séchée et broyée, sous forme de poudre fine et homogène, ont été mélangés à 60 kg d'huile d'avocat et 20 kg d'huile de rosier muscat (rapport massique huile d'avocat/huile de rosier muscat = 3/1). Le rapport massique plante / huile était de 0,20.

Le mélange a ensuite été chauffé rapidement par micro-ondes à une puissance de 10000 à 30000 Watts/ kg de mélange, pendant une durée inférieure à 10 minutes, sous flux d'azote, la température maximale atteinte étant comprise entre 80 et 200°C.

Le mélange a ensuite été traité par ultrasons (100 kHz environ), sous azote et sous agitation, pendant 10 à 20 minutes environ. Le mélange d'huiles vectrices contenant l'extrait lipophile d'*Hemerocallis fulva* a été séparé par centrifugation.

### EXEMPLE 2 : Mise en évidence de l'absence de cytotoxicité de la composition huileuse conforme à l'invention

Dans cet exemple on a testé la cytotoxicité éventuelle de la composition huileuse conforme à l'invention, telle qu'obtenue par le procédé de l'Exemple 1.

Le test sur la viabilité d'épidermes humains reconstruits utilisé est celui de la réduction au bleu de Formazan (test MTT). L'essai a été réalisé *in vitro* sur épidermes humains reconstruits (« *Reconstructed Human Epidermis* », RHE) modèle RHEps, de surface 0,5 cm², vendus par la société SkinEthic ®.

Le mélange d'huiles vectrices (huile d'avocat/huile de rosier muscat) dans le rapport massique 3/1 tel qu'utilisé pour l'extraction réalisée à l'exemple 1 a été testé pur (100 %), en application topique sur les épidermes.

La composition huileuse telle que préparée à l'exemple 1 a été testée après dilution à 0,5% (v/v), 1% (v/v), 1,5% (v/v) et 3% (v/v) dans de l'huile de paraffine. Chacune des compositions huileuses ainsi diluées a été appliquée à raison de 5 mg/cm², sur le stratum corneum des épidermes.

Les mesures de viabilité tissulaires par test MTT ont été effectués après 24 heures d'incubation à 37°C en atmosphère humide air-CO₂ (35%/5%), un épiderme non traité servant de témoin.

Les résultats sont reportés dans le tableau 1 ci-après.

**TABLEAU I**

| **Substance** | **Densité optique à 540 nm** | **Viabilité (%)** |
|---|---|---|
| Aucune (Témoin) | 1,058 ± 0,007 | 100 |
| Huiles vectrices pures | 1,058 ± 0,010 | 100 |
| Composition huileuse à 0,5 % | 1,048 ± 0,013 | 99 |
| Composition huileuse à 1 % | 1,057 ± 0,007 | 100 |
| Composition huileuse à 1,5 % | 1,050 ± 0,006 | 99 |
| Composition huileuse à 3,0 % | 1,031± 0,007 | 97 |

Ces résultats montrent qu'aucune diminution significative de la viabilité cellulaire n'est enregistrée après traitement topique avec la composition huileuse conforme à la présente invention dans la gamme de concentrations testées ; ceci comparativement au mélange des deux huiles vectrices testées dans les mêmes conditions qui ne présente lui non plus, aucun effet toxique vis-à-vis des tissus cutanés.

### EXEMPLE 3 : Mise en évidence des propriétés de la composition huileuse selon l'invention sur l'amélioration de la fermeté de la peau

Dans cet exemple, on a évalué les effets de deux compositions conformes à l'invention se présentant sous forme de crème sur l'amélioration de la fermeté de la peau, lesdites compositions comprenant des quantités différentes de la composition huileuse selon l'invention, à savoir :
- Composition huileuse telle que préparée à l'exemple 1, formulée à 1 % en masse dans une crème pour le visage (ci-après dénommée « Crème à 1 % ») ;
- Composition huileuse telle que préparée à l'exemple 1, formulée à 4 % en masse dans une crème pour le visage (ci-après dénommée « Crème à 4 % »).

La formulation de la crème à 1 % était la suivante :
- Eau 92,9 %
- Huile de tournesol 4 %
- Copolymère d'ammonium acryloyldiméthyltaurate et de vinylpyrrolidone vendu sous la dénomination commerciale Aristoflex® AVC par Clariant 2 %
- Conservateur 1%
- Composition huileuse de l'exemple 1 1 %

La formulation de la crème à 4 % était en tout point identique à celle de la crème à 1 % ci-dessus sauf qu'elle contenait 4 % en masse de la composition huileuse de l'exemple et seulement 89,9 % en masse d'eau.

### 3.1. Principe des tests

Par ailleurs, l'évaluation des crèmes vis-à-vis de l'amélioration de la fermeté de la peau a été effectuée sur deux indicateurs :
i) la fibrilline afin de déterminer la capacité des crèmes conformes à l'invention à prévenir la dégradation de la fibrilline, celle-ci ayant un rôle dans le maintien de l'élasticité du derme ;
ii) les filaments de vimentine afin de déterminer la capacité des crèmes conformes à l'invention à prévenir une perte d'activité ou une dégradation de la vimentine, ceux-ci étant impliqués dans l'organisation de la contraction et de la cohésion du derme en permettant aux fibroblastes de conserver une bonne contractilité.

### 3.2. Protocoles

### 3.2.1 Préparation des coupes histologiques

L'étude a été réalisée sur des explants de peau humains d'un diamètre de 10 mm préparés à partir d'une plastie abdominale d'une femme âgée de 53 ans.

Un nombre suffisant d'explants pour réaliser l'ensemble des tests décrits ci-après ont été prélevés. A réception, les explants ont été transférés dans des plaques de 12 puits contenant du milieu de culture pour épiderme vendu par la société SkinEthic (0,5 ml/puits) et placés à l'incubateur à 37°C, dans une atmosphère à 5% de CO₂ saturée d'humidité, pendant 24 heures avant de procéder au traitement des tissus.

Les explants ont ensuite subi différents traitements, chaque traitement ayant été réalisé en triplicate :
**Lot TEMOIN T0** : Aucun traitement, prélèvement à 0 jours ;
**Lot TEMOIN J9** : Aucun traitement, prélèvement à 9 jours ;
**Lot Crème à 1 %** : traitement avec la crème à 1 %, prélèvement à 9 jours ;
**Lot Crème à 4 %** : traitement avec la crème à 4 %, prélèvement à 9 jours ;
**Lot MG :** Induction de la glycation par le méthylglyoxal, aucun traitement, prélèvement à 9 jours ;
**Lot Crème à 1 % + MG :** Induction de la glycation par le méthylglyoxal, traitement avec la crème à 1 %, prélèvement à 9 jours ;
**Lot Crème à 4 % + MG :** Induction de la glycation par le méthylglyoxal, traitement avec la crème à 4 %, prélèvement à 9 jours.

### Application des produits :

Aux jours J0, J2, J3 et J6, les produits (crème à 1% ou crème à 4 %) ont été appliqués sur les explants à raison de 2 mg de crème par explant. Les explants des lots TEMOIN J9 n'ont reçu aucun traitement. Les milieux de culture ont été renouvelés aux jours J3 et J6.

### Induction de la glycation :

Aux jours J3 et J6, 18 µl d'une solution de méthylglyoxal à 55 mM dans l'eau stérile (cette solution a été préparée à partir d'une solution commerciale de méthylglyoxal Sigma M0252 à 40%) ont été ajoutés aux puits contenant les explants des lots **MG ; Crème à 1 % + MG et Crème à 4 % + MG.** La concentration finale en MG dans chacun des puits était de 500 µM.

### Prélèvements des explants :

A J0, les explants du lot TEMOIN T0 ont été prélevés et coupés en deux. Une moitié a été fixée par du formol tamponné pendant 24 heures et l'autre moitié a été congelée à - 80°C.

A J9, les 3 explants de chacun des lots ont été prélevés et traités de la même façon.

### Traitement histologique des explants

Après 24 heures de fixation dans le formol tamponné, les prélèvements ont été déshydratés et imprégnés en paraffine à l'aide d'un automate de déshydratation Leica ® TP 1010.

Les prélèvements ont ensuite été mis en bloc de paraffine à l'aide d'une station d'enrobage Leica ® EG 1160.

Des coupes de 5 µm ont été réalisées à l'aide d'un microtome type Minot, Leica ® RM 2125, puis fixées et montées sur des lames de verre histologiques.

Les coupes histologiques ont été ensuite été déparaffinées dans des bains de xylène et rincées à l'alcool absolu.

### Immuno-marquage de la Fibrilline-1 :

La fibrilline-1 a ensuite été marquée avec un anticorps monoclonal de souris anti-fibrilline-1 (clone 11.C1.4, vendu par la société Santa Cruz Biotechnology Inc. sous la référence sc-66058), au 1/100^{ème} pendant 1 heure à température ambiante à l'aide d'un automate d'immunomarquage Autostainer Plus® vendu par la société Dako. Les lames ont ensuite été rincées au tampon phosphate salin (PBS pH 7,4 ; 2 x 5 min) puis immergées pendant 2 x 30 minutes dans un complexe avidine-biotine (système amplificateur) vendu sous la dénomination commerciale R.T.U. VECTASTAIN® ABC Elite® Kit (société Vector Laboratories), rincées 2 x 5min au PBS et enfin révélées par mise en contact avec de l'isothiocyanate de fluorescéine (Fluorescéine IsoThioCyanate : FITC - SA 1002, Caltag) pendant 30 minutes à température ambiante. Pour finir, les lames ont encore été rincées 2 x 5 min au PBS.

Les noyaux ont été contre colorés par mise en contact des lames avec une solution d'iodure de propidium à 0,2% de la solution mère à 0,1 mg/ml dans du PBS pH 7,4 pendant 2 minutes, puis rincées au PBS 2 x 5 min puis à l'eau distillée pendant 5 minutes.

Après séchage à température ambiante, les lames ont ensuite été observées avec un microscope optique, les prises de vue ont été réalisées avec une caméra Olympus DP72 ® et le logiciel Cell-D.

Après immuno-marquage, le marquage de fibrilline apparait en vert fluorescent, ce qui correspond à un gris clair sur une photo en noir et blanc.

### Immuno-marquage des filaments de vimentine :

La vimentine a été marquée avec une solution d'anticorps de souris anti-vimentine (clone V9, Référence sc-6260 vendu par la société Santa-Cruz Biotechnology Inc.) dilué au 1/400^{ème} dans une solution d'albumine de sérum bovin (BSA pour « *Bovine Serum Albumine* ») à 3 mg/ml dans du PBS.

Les lames supportant les coupes histologiques déparaffinées ont été mises en contact avec cette solution d'anticorps pendant 2 heures à température ambiante. Après rinçage avec du PBS pH 7,4, les lames ont été immergées pendant 2 x 30 minutes dans un complexe avidine-biotine (système amplificateur) vendu sous la dénomination commerciale R.T.U. VECTASTAIN® ABC Elite® Kit (société Vector Laboratories), puis rincées au PBS 2 x 5 min et enfin révélées par mise en contact avec un kit à la peroxydase pendant 4 min vendu sous la dénomination Vector SK-4600 par la société Vector Laboratories. Après rinçage à l'eau distillée et déshydratation, les lames ont été montées à l'aide du milieu de montage vendu sous la dénomination commerciale Eukitt® par la société Merck.

### Tests semi-quantitatifs

Les études des coupes histologiques ont été complétées par la réalisation d'un test semi-quantitatif sur les lots TEMOIN T0, TEMOIN J9, Crème à 1%, MG et Crème à 1 % + MG afin de déterminer la surface occupée par la fibrilline au niveau du derme papillaire ainsi que la surface occupée par la fibroblastes et leur morphologie.

La surface occupée par la fibrilline au niveau du derme papillaire et par les fibroblastes a été mesurée par analyse d'image grâce au logiciel QWin® de la société Leica. Ce logiciel permet de mesurer différents paramètres morphologiques tels que la surface, la circularité (pour les fibroblastes, plus ou moins ronds ou fusiformes) ou encore le nombre de fourches d'une cellule (témoin de la dendricité, dans ce cas dendricité du fibroblaste, et témoignant de la bonne santé et activité de la cellule).

8 à 12 photos par lot ont été analysées.

La circularité des fibroblastes a également été déterminée. Ce paramètre est calculé en fonction du rapport du périmètre carré de la surface. Pour un objet parfaitement rond, la circularité est égale à 1, quelle que soit la taille de l'objet. Plus cette valeur est proche de 1, plus la cellule est ronde, donc en souffrance dans le cas du fibroblaste dont la forme, à l'état normal, est fusiforme.

Le nombre de fourches des fibroblastes (dendricité) a également été déterminé de façon visuelle à l'aide du logiciel QWin® de la société Leica qui permet de faire de la morphométrie et de mesurer visuellement la présence ou non de fourches ainsi que la circularité d'une cellule.

### 3.3. Résultats

### 3.3.1 : Résultats relatifs à la fibrilline

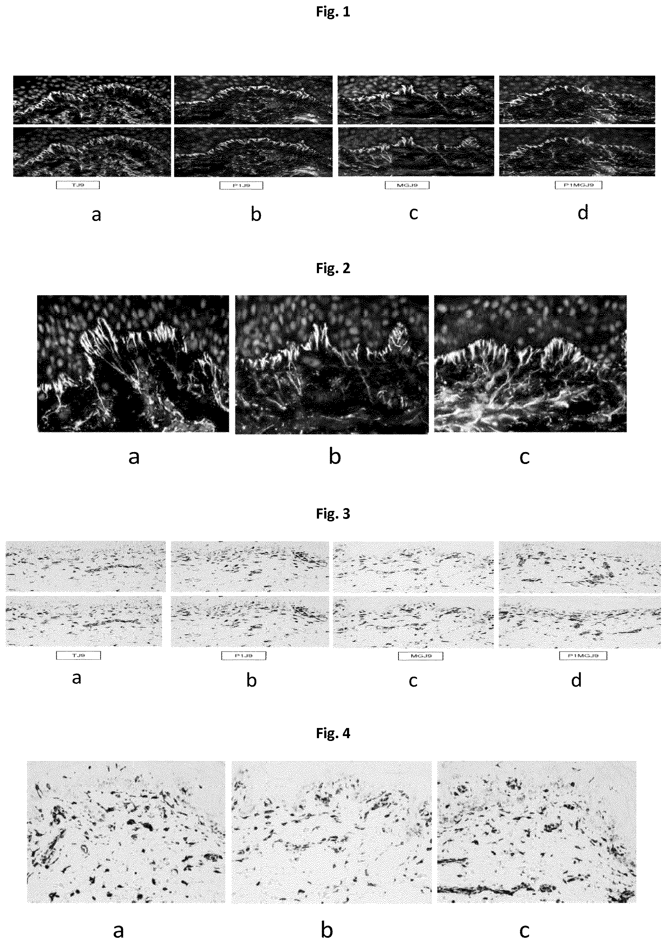

Les photos des coupes des explants correspondant à chacun des lots sont données sur les figures 1 et 2 annexées :
- Figure la : coupe d'un explant du lot TEMOIN J9 ;
- Figure 1b : coupe d'un explant du lot Crème à 1 % ;
- Figure 1c : coupe d'un explant du lot MG ;
- Figure 1d : coupe d'un explant du lot Crème à 1 % + MG ;
- Figure 2a : coupe d'un explant du lot Crème à 4 % ;
- Figure 2b : coupe d'un explant du lot MG ;
- Figures 2c : coupe d'un explant du lot Crème à 4 % + MG.

En absence de traitement et de stress glyquant (photo de la figure la), on note que la fibrilline est présente à la fois dans le derme (constituant de l'élastine) et au niveau de la jonction dermo-épidermique (fibres d'oxytalane allant du derme à la JDE).

On constate que l'application de la crème formulée avec la composition huileuse à 1% en absence de stress glyquant n'induit pas de variation de l'expression de la fibrilline-1 (photo de la figure 1b).

Après incorporation du MG dans le milieu de culture et comparativement au lot témoin à J9 **(TEMOIN J9),** on observe une diminution de l'expression de la fibrilline-1, notamment au niveau des fibres d'oxytalane. Le MG a donc une action significative négative sur la synthèse de la fibrilline-1 (photos de la figure 1c et 2b).

L'application de la crème à 1% après que l'explant ait été mis en contact avec MG (stress glyquant) induit une augmentation de l'expression de la fibrilline-1 et compense donc les effets néfastes du MG (Photo de la figure 1d).

L'application de la crème à 4% après soumission de l'explant à un stress glyquant, induit une augmentation assez importante de l'expression de la fibrilline-1 et compense donc également les effets néfastes du MG, de façon sensiblement équivalente à la crème à 1 % (Photo de la figure 2c). En cas de stress glyquant, la composition huileuse utilisée conformément à l'invention agit donc comme un véritable protecteur et non pas comme un stimulateur de la synthèse de la fibrilline-1.

Les résultats du test semi-quantitatif de la surface occupée par la dityrosine sont donnés dans le tableau II ci-après :

**TABLEAU II**

| **Lot** | **Surface occupée par la fibrilline-1 (en %)** | |
|---|---|---|
| | **Moyenne** | **Ecart type** |
| **TEMOIN T0** | 33,8 | 7,2 |
| **TEMOIN J9** | 42,6 | 4,0 |
| **Crème à 1 %** | 38,6 | 5,5 |
| **MG** | 35,0 | 3,1 |
| **Crème à 1 % + MG** | 43,5 | 5,4 |

Il ressort de ces résultats qu'à J0 (TEMOIN T0) la fibrilline-1 occupe 33,8 % de la surface du derme papillaire. Au bout de 9 jours, sur le lot non traité (TEMOIN J9), l'expression de la fibrilline-1 est significativement augmentée de 26 % comparativement au lot TEMOIN T0.

Après application de la crème à 1 % et au bout de 9 jours de traitement (Lot Crème à 1%), on observe une diminution non significative de 9 % de la surface du derme papillaire occupée par la fibrilline-1. On peut donc en conclure que l'application de la composition huileuse formulée à 1 % dans une crème sur une peau non stressée n'a pas d'activité significative. Ces résultats confirment ce qui a été constaté sur les photos (figures la à 1c).

Après incorporation du MG dans le milieu de culture, et en l'absence de traitement (Lot MG), l'expression de la fibrilline-1 est significativement diminuée de 18 %.

Comparativement au lot traité avec du MG seul (Lot MG), et après 9 jours de traitement, l'application de la crème formulée à 1 % en masse de composition huileuse après stress glyquant (Lot Crème à 1 % + MG) induit une augmentation significative de 24 % de l'expression de la fibrilline-1 dans le derme papillaire. L'application de la crème formulée à 1 % en masse en composition huileuse inhibe donc totalement les effets néfastes du stress glyquant.

### Conclusion

L'ensemble de ces résultats relatifs à la fibrilline-1 met en évidence qu'en présence de la composition huileuse utilisée conformément à l'invention, les effets du composé glyquant sont diminués, jusqu'à retrouver une petite synthèse de cette protéine, même si clairement le rôle la composition huileuse est protecteur.

On peut donc considérer que la composition huileuse formulé à 1% et 4% permet de maintenir la qualité et la quantité de la fibrilline-1 et donc la fonctionnalité de l'élastine. La qualité de l'élastine étant maintenue, on peut donc aisément en déduire que l'on préserve la fermeté de la peau malgré l'apparition avec l'âge, de produits glyquants.

Les éléments chiffrés confirment très clairement qu'en cas de stress de la peau (stress glyquant), la composition huileuse est capable de compenser ces effets néfastes en totalité.

### 3.3.2 : Résultats relatifs à la vimentine

Remarques préliminaires à l'exploitation des résultats : Une diminution de l'intensité de la fluorescence ne doit pas forcément être interprétée comme étant liée à une perte d'activité ou à une dégradation de la vimentine. En effet, lorsque les filaments de vimentine sont stimulés, ils entrainent la formation de dendrites, c'est-à-dire de prolongements cellulaires qui vont permettre aux fibroblastes de multiplier leurs points d'attache avec la matrice extra cellulaire (MEC), notamment avec le réseau de fibres de collagène et d'élastine. A l'inverse, lorsque la cellule est soumise à un stress « glyquant » (ici induit par le MG), la vimentine est altérée et n'est plus capable d'exercer son rôle de maintien et d'organisation de la MEC. Les filaments restants se concentrent alors au niveau du noyau. Cette concentration entrainée par la glycation peut être responsable d'une augmentation de l'intensité de fluorescence.

Les photos des coupes des explants correspondant à chacun des lots sont données sur les figures 3 et 4 annexées :
- Figure 3a : coupe d'un explant du lot TEMOIN J9 ;
- Figure 3b : coupe d'un explant du lot Crème à 1 % ;
- Figure 3c : coupe d'un explant du lot MG ;
- Figure 3d : coupe d'un explant du lot Crème à 1 % + MG ;
- Figure 4a : coupe d'un explant du lot Crème à 4 % ;
- Figure 4b : coupe d'un explant du lot MG ;
- Figures 4c : coupe d'un explant du lot Crème à 4 % + MG.

En absence de traitement et de stress glyquant (photo de la figure 3a), on note que le marquage de la vimentine est assez net au niveau des fibroblastes, de plus la dendricité est assez développée, ce qui traduit de la bonne santé des fibroblastes.

On constate que l'application de la crème formulée avec la composition huileuse à 1% en absence de stress glyquant entraine une augmentation modérée de la vimentine au niveau des fibroblastes, ainsi qu'au niveau de leur dendricité (photo de la figure 3b). On constate donc que la composition huileuse formulée à 1 % en masse dans une crème, dans des conditions normales d'application, n'induit pas de réaction particulière.

L'application de la crème formulée à 4 % en masse de composition huileuse entraine une nette augmentation de l'expression de la vimentine au niveau des fibroblastes et une augmentation modérée de la dendricité (photo de la figure 4a).

Après incorporation du MG dans le milieu de culture et comparativement au lot témoin à J9 **(TEMOIN J9),** on observe une dendricité plus faible des fibroblastes traduisant d'un ralentissement de leur activité et d'une altération des filaments de vimentine (photos de la figure 3c et 4b).

Lorsque l'on applique la crème à 1% après que l'explant ait été mis en contact avec MG (stress glyquant), on observe une très nette augmentation de la dendricité des fibroblastes traduisant d'une reprise de leur activité ainsi qu'une stimulation de la vimentine (Photo de la figure 1d). En effet, lorsque les filaments de vimentine sont stimulés, ils entrainent la formation de dendrites, c'est-à-dire de prolongements cellulaires qui vont permettre aux fibroblastes de multiplier leurs points d'attache avec la MEC, notamment avec le réseau de fibres de collagène et d'élastine.

L'application de la crème à 4% après soumission de l'explant à un stress glyquant, induit également une nette augmentation de la dendricité traduisant également d'une reprise d'activité du fibroblaste (Photo de la figure 2c).

Les résultats du test semi-quantitatif de la surface occupée par les fibroblastes sont donnés dans le tableau III ci-après :

**TABLEAU III**

| **Lot** | **Surface occupée par les fibroblastes (en µm²)** | |
|---|---|---|
| | **Moyenne** | **Ecart type** |
| **TEMOIN T0** | 118,4 | 47,9 |
| **TEMOIN J9** | 122,9 | 50,4 |
| **Crème à 1 %** | 134,6 | 56,5 |
| **MG** | 137,1 | 56,9 |
| **Crème à 1 % + MG** | 129,3 | 55,4 |

Il ressort de ces résultats qu'à J0 (TEMOIN T0) les fibroblastes présentent une surface moyenne de 118,4 µm². Au bout de 9 jours, sur le lot non traité (TEMOIN J9), la surface des fibroblastes est augmentée de façon non significative de 3,8 % comparativement au lot TEMOIN T0.

Après application de la crème à 1 % et au bout de 9 jours de traitement (Lot Crème à 1%), on observe une augmentation significative de 9,5 % de la surface des fibroblastes.

Après incorporation du MG dans le milieu de culture, et en l'absence de traitement (Lot MG), la surface des fibroblastes est significativement augmentée de 11,5 % (le MG réduit la dendricité et concentre le cytoplasme autour du noyau, ce qui augmente la surface mesurée).

Comparativement au lot traité avec du MG seul (Lot MG), et après 9 jours de traitement, l'application de la crème formulée à 1 % en masse de composition huileuse après stress glyquant (Lot Crème à 1 % + MG) induit une diminution significative de 5,7 % de la surface des fibroblastes.

Le tableau IV ci-après donne les résultats relatifs à la mesure de la circularité des fibroblastes :

**TABLEAU IV**

| **Lot** | **Circularité des fibroblastes** | |
|---|---|---|
| | **Moyenne** | **Ecart type** |
| **TEMOIN T0** | 3,19 | 1,55 |
| **TEMOIN J9** | 3,11 | 2,09 |
| **Crème à 1 %** | 3,33 | 1,66 |
| **MG** | 2,54 | 1,38 |
| **Crème à 1 % + MG** | 3,49 | 1,77 |

Il ressort de ces résultats qu'à J0 (TEMOIN T0) les fibroblastes présentent une circularité de 3,19. Les fibroblastes sont dont bien fusiformes.

Au bout de 9 jours, sur le lot non traité (TEMOIN J9), la circularité des fibroblastes est augmentée de façon non significative de 2,5 % comparativement au lot TEMOIN T0. Les fibroblastes sont donc toujours fusiformes.

Après application de la crème à 1 % et au bout de 9 jours de traitement (Lot Crème à 1%), on observe une diminution non significative de 7,1 % de la circularité des fibroblastes. Il n'y a donc pas d'évolution de la forme des fibroblastes.

Après incorporation du MG dans le milieu de culture, et en l'absence de traitement (Lot MG), la circularité des fibroblastes est significativement augmentée de 18,4 % ce qui signifie que les fibroblastes ont perdu leur caractère fusiforme pour s'arrondir. Les fibroblastes sont par conséquent devenus moins fonctionnels et ont perdu leurs points d'ancrage.

Comparativement au lot traité avec du MG seul (Lot MG), et après 9 jours de traitement, l'application de la crème formulée à 1 % en masse de composition huileuse après stress glyquant (Lot Crème à 1 % + MG) induit une diminution significative de 37,7 % de la circularité des fibroblastes. L'application de cette crème permet ainsi d'inhiber totalement les effets néfastes du stress glyquant.

Les résultats relatifs au nombre de fourches des fibroblastes sont donnés dans le tableau V ci-après :

**TABLEAU V**

| **Lot** | **Nombre de fourches moyen par fibroblaste** | |
|---|---|---|
| | **Moyenne** | **Ecart type** |
| **TEMOIN T0** | 1,70 | 1,51 |
| **TEMOIN J9** | 1,86 | 1,66 |
| **Crème à 1 %** | 1,94 | 1,69 |
| **MG** | 1,40 | 1,11 |
| **Crème à 1 % + MG** | 1,90 | 1,59 |

Il ressort de ces résultats qu'à J0 (TEMOIN T0) les fibroblastes présentent en moyenne 1,7 fourches.

Au bout de 9 jours, sur le lot non traité (TEMOIN J9), le nombre de fourches par fibroblaste est augmenté de façon non significative de 9,5 % comparativement au lot TEMOIN T0.

Après application de la crème à 1 % et au bout de 9 jours de traitement (Lot Crème à 1%), on n'observe pas d'augmentation significative (4,5 %) du nombre de fourches des fibroblastes par rapport au témoin.

Après incorporation du MG dans le milieu de culture, et en l'absence de traitement (Lot MG), le nombre de fourches des fibroblastes est significativement diminué de 24,4 % ce qui signifie que les fibroblastes ont été altérés.

Comparativement au lot traité avec du MG seul (Lot MG), et après 9 jours de traitement, l'application de la crème formulée à 1 % en masse de composition huileuse après stress glyquant (Lot Crème à 1 % + MG) induit une augmentation significative de 35,5 % du nombre de fourches des fibroblastes. L'application de cette crème permet ainsi d'inhiber totalement les effets néfastes du stress glyquant et la restauration des fibroblastes.

### 3.3.3 : Conclusions

L'ensemble de ces résultats démontre que la composition huileuse comprenant l'extrait lipophile d'hémérocalle utilisée conformément à l'invention possède une excellente activité anti-glycation très marquée dès 1% et qui se confirme à 4% :
- sur la protection de la fibrilline. En effet la fibrilline n'est pas altérée en présence de l'extrait lipophile d'hémérocalle suite à un stress glyquant, elle est protégée, on remarque même une augmentation d'expression de cette protéine de 24% ;
- sur la détoxification des fibres de vimentine (cytosquelette des fibroblastes) ainsi que sur l'amélioration des propriétés contractiles des fibroblastes (formation de dendrites), témoignant de leur bonne santé.

Les résultats obtenus sur coupe de peau sont confirmés par les résultats chiffrés qui attestent bien l'activité de l'extrait lipophile d'hémérocalle sur la fibrilline et la vimentine lors d'un stress glyquant.

On démontre ainsi une réelle protection du fibroblaste vis-à-vis de la glycation de la vimentine qui fait partie intégrante du cytosquelette du fibroblaste. On peut conclure que l'on maintient sa structure et donc son métabolisme en contrant les effets de la glycation grâce à l'application d'une crème renfermant une composition huileuse à base d'un extrait lipophile d'hémérocalle.

Ce point est capital quand on souhaite parler de fermeté puisque le fibroblaste est le siège de la synthèse des glycosaminoglycanes, de l'élastine, des collagènes... Lui permettre de fonctionner de façon optimale permet de maintenir une bonne qualité et quantité des éléments produits. Ces différents éléments participent au soutien du derme et donc à la fermeté de la peau.

De plus, il a été démontré que la fibrilline est également protégée contre la glycation, or cette protéine rentre dans la composition de l'élastine, elle est donc directement impliquée dans la fermeté et l'élasticité de la peau.

Ces deux éléments réunis montrent que l'extrait lipophile d'hémérocalle est un actif important dans le maintien de la fermeté au cours du vieillissement car il est capable d'enrayer les effets nocifs des AGEs.

### EXEMPLE 4 : Etudes cliniques

### 4.1. Etude sensorielle

Une étude clinique a été réalisée de façon strictement confidentielle sur un panel de 270 volontaires afin d'assoir l'allégation « amélioration de l'éclat du teint » sur une période de 28 jours. Il est à noter que sur les 270 volontaires, seuls 256 ont effectué l'étude dans sa durée totale. La composition des formules n'était pas mentionnée sur les emballages et n'a pas été communiquée aux membres du panel.

Elle a été réalisée sur des produits de soin appartenant à la gamme vendue sous la dénomination commerciale Merveillance® par la société Nuxe, dans lesquels a été ajouté 1 % en masse de composition huileuse telle que préparée ci-dessus à l'exemple 1.

Les références testées de la Gamme Merveillance® sont les suivantes :
- Crème Peaux Sèches à Très Sèches (PS-TS),
- Crème Peaux Normales à Sèches (PN-PS),
- Crème Peaux Normales à Mixtes (PN-PM),
- Crème de nuit Peaux Normales à Sèches (Nuit-PN-PS),
- Contour des yeux (CY),
- Sérum.

Chacun de ces produits commerciaux a été modifié en y ajoutant 1 % en masse d'une composition huileuse telle que préparée ci-dessus à l'exemple 1.

Pour cette étude, les volontaires ont été divisés en 4 groupes :
- Groupe 1 (G1) : Sérum + CY + PN-PS,
- Groupe 2 (G2) : Sérum + CY + Nuit-PN-PS,
- Groupe 3 (G3) : Sérum + CY + PS-TS,
- Groupe 4 (G4) : Sérum + CY + PN-PM.

Les produits ont été appliqués selon les recommandations suivantes :
Sérum + contour des yeux : une application le matin ;
Les crèmes (PS-TS ; PN-PS ; PN-PM) : une application le matin et une application le soir.

Pendant la période du test, les membres du panel n'ont appliqué aucun autre produit cosmétique que ceux testés sur leur peau.

Le tableau VI ci-après regroupe les traitements reçus en fonction du nombre de volontaires :

**TABLEAU VI**

| **Nombre de volontaires** | **Sérum** | **CY** | **PN-PS** | **Nuit-PN-PS** | **PS-TS** | **PN-PM** |
|---|---|---|---|---|---|---|
| **à J0** | 270 | 270 | 68 | 68 | 67 | 67 |
| **à J28** | 256 | 261 | 66 | 63 | 63 | 65 |

Il a été demandé à chaque volontaire, à J0 et à J28, de répondre par « oui » ou par « non » à la question suivante : votre peau vous semble-t-elle raffermie ?

Les réponses des membres du panel (en %), en fonction des groupes, sont reportées dans le tableau VII ci-après :

**TABLEAU VII**

| **Temps** | **G1** | **G2** | **G3** | **G4** |
|---|---|---|---|---|
| **à J0** | 49% | 53 % | 58% | 52% |
| **à J28** | 63% | 72% | 61 % | 71 % |

Au vu de ces résultats, le nombre de testeurs étant conséquent, on peut conclure que l'application des produits comprenant 1 % en masse de composition huileuse conforme à l'invention conduit bien à un gain de fermeté.

### 4.2. Etudes des propriétés biomécaniques

Un test complémentaire visant à étudier les propriétés biomécaniques de la peau a également été effectué.

Ce test a été réalisé sur 32 personnes choisies parmi les membres du groupe 3 (peaux sèches à très sèches), après application de la crème PS-TS pendant 28 jours. Les mesures ont été réalisées à J0 et au bout de 28 jours à l'aide de l'appareil Dynaskin® (société EOTECH) couplé à l'appareil DermaTOP ® (société EOTECH).

Cet appareillage permet de mesurer la fermeté de la peau dans des conditions très reproductibles. L'étude se déroule en trois mesures consécutives:
- la première mesure acquiert la zone d'intérêt avant déformation de la peau au niveau de la joue ;
- la deuxième mesure acquiert la zone d'intérêt pendant la déformation qui est créée par un jet d'air formant un angle de 90° par rapport à la surface de la peau au niveau de la joue ;
- la troisième mesure acquiert la zone d'intérêt après la déformation de la peau au niveau de la joue.

La pression du jet d'air a été réglée à 1,8 g et la durée de déformation était de 5 à 6 secondes.

Un logiciel dédié calcule ensuite à J0 et à J28, le volume, la surface et la profondeur de la déformation. Il est ensuite possible de calculer les % de variation du volume, de la surface et de la profondeur entre les mesures effectuées à J0 et à J28 et ainsi évaluer l'efficacité de la composition sur l'amélioration de la fermeté de la peau.

Les résultats, exprimés en % différentiels à J28 par rapport aux mesures effectuées à J0 moyens figurent dans le tableau VIII ci-après :

**TABLEAU VIII**

| | **Evolution à J28 par rapport à J0 (en %)** |
|---|---|
| **Volume de la déformation** | - 10,7 % |
| **Surface** | +3,7% |
| **Profondeur** | - 10,9 % |

On constate que la crème PS-TS contenant 1 % de composition huileuse conforme à l'invention induit, par rapport à J0, une diminution significative du paramètre profondeur de 10,9 %, ainsi que paramètre volume de 10,7 %.

### EXEMPLE 5 : Crème contour des yeux fermeté

Par les techniques usuelles, on a préparé une crème légère contour des yeux ayant la composition massique indiquée ci-après.
- EDTA tetrasodique 0,1 g
- Caféine 0,2 g
- Glycérine 3,0 g
- Mélange composé d'un copolymère hydroxyéthyle acrylate/sodium acryloyl-diméthyl taurate (SEPINOV EMT10 ; société SEPPIC), d'isostéarate de Sorbitan et de Polysorbate 60 1,0 g
- Huile de rosier muscat 3,0 g
- Oléate de décyle 3,0 g
- Tocophérol 0,7 g
- Composition huileuse de l'exemple 1 1,0 g
- Système de conservation 0,8 g
- Parfum 0,5 g
- Eau qsp 100,0 g

Ce contour des yeux peut être utilisé une à deux fois par jour pendant une durée de 1 à 6 mois.

### EXEMPLE 6 : Crème peau sèche fermeté

Par les techniques usuelles, on a préparé une crème pour peaux sèches destinée à améliorer la fermeté de la peau et ayant la composition massique suivante :
- EDTA tetrasodique 0,1 g
- Caféine 0,2 g
- Glycérine 3,0 g
- Polymère réticulé d'acrylate/C₁₀₋₃₀ alkyle
   acrylate vendu sous la dénomination commerciale
   Carbopol® Ultrez 21 par Gattefossé 1,0 g
- Mélange d'alcool arachidylique, d'alcool
   behénique et de glucoside d'arachidyle vendu sous la dénomination Montanov® 202 par SEPPIC 4,0 g
- Huile de noisette 6,0 g
- Beurre de karité 2,0 g
- Oléate de décyle 3,0 g
- Tocophérol 0,7 g
- Composition huileuse de l'exemple 1 2,0 g
- Système de conservation 0,8 g
- NaOH 0,08 g
- Parfum 0,5 g
- Eau qsp 100,0 g

Cette crème peut être utilisée une à deux fois par jour par application sur les zones de la peau à traiter (visage).

### EXEMPLE 7 : Sérum fermeté

Par les techniques usuelles, on a préparé un sérum destiné à améliorer la fermeté de la peau ayant la composition massique suivante :
- EDTA tetrasodique 0,1 g
- Extrait de plancton vendu sous la dénomination EPS Seafill par la société CODIF 3,0 g
- Glycérine 3,0 g
- Carbomer vendu sous la dénomination Carbopol® Ultrez 10 par la société Gattefossé 0,2 g
- Glutamate de stéaryle sodique 0,5 g
- Huile de rosier muscat 2,0 g
- Oléate de décyle 1,0 g
- Copolymère réticulé à base de polydiméthysiloxane vendu sous la dénomination commerciale DC9041 par la société Dow Corning 1,0 g
- Tocophérol 0,7 g
- Composition huileuse de l'exemple 1 5,0 g
- Hyaluronate de sodium 0,3 g
- Soude 0,15 g
- Système de conservation 0,8 g
- Parfum 0,5 g
- Eau qsp 100,0 g

Ce sérum peut être utilisé une à deux fois par jour par application sur les zones de la peau à traiter (visage).

## Revendications

1. Utilisation, à titre d'ingrédient actif, d'une composition huileuse à base d'un extrait lipophile *d'Hemerocallis fulva* et d'un véhicule huileux comprenant au moins une huile végétale, pour la préparation d'une composition cosmétique à usage topique destinée à améliorer la fermeté et/ou l'élasticité de la peau.

2. Utilisation selon la revendication 1, **caractérisée en ce que** la composition huileuse représente de 0,1 à 10 % en masse par rapport à la masse totale de la composition cosmétique.

3. Utilisation selon la revendication 1, **caractérisée en ce que** la composition huileuse représente de 0,5 à 4 % en masse par rapport à la masse totale de la composition cosmétique.

4. Utilisation selon l'une quelconque des revendications précédentes, **caractérisée** en ledit extrait est obtenu à partie des parties aériennes *d'Hemerocallis fulva.*

5. Utilisation selon l'une quelconque des revendications précédentes, **caractérisée en ce que** ledit extrait est obtenu par extraction à l'abri de l'air.

6. Utilisation selon l'une quelconque des revendications précédentes, **caractérisée en ce que** le véhicule huileux comprend au moins une huile végétale naturelle choisie parmi l'huile d'avocat, l'huile de rosier muscat, l'huile de noisette, l'huile de colza, l'huile de macadamia, l'huile d'argan, l'huile de tournesol, l'huile de cameline, l'huile de bourrache, l'huile d'amande douce, l'huile de carthame, l'huile de calophyllum et leurs mélanges.

7. Utilisation selon la revendication 6, **caractérisée en ce que** l'huile végétale naturelle est choisie parmi l'huile d'avocat, l'huile de rosier muscat et leurs mélanges.

8. Utilisation selon la revendication 7, **caractérisée en ce que** le véhicule huileux comprend un mélange d'huile d'avocat et d'huile de rosier muscat.

9. Utilisation selon la revendication 8, **caractérisée en ce que** le rapport massique huile d'avocat/huile de rosier muscat au sein de la composition huileuse varie de 4/1 à 1/1.

10. Utilisation selon l'une quelconque des revendications précédentes, **caractérisée en ce que** la teneur en extrait lipophile d'hémérocalle varie de 5 à 20 ppm.

11. Utilisation selon l'une quelconque des revendications précédentes, **caractérisée** en ce la composition cosmétique contient en outre un ou plusieurs actifs secondaires choisis parmi un agent anti-âge, l'acétyl tetrapeptide-5, un extrait de plancton, un extrait de maca, un extrait de pétales de coquelicot, une combinaison d'extraits d'anchuse, de coquelicot et de passiflore, un extrait de graines de mimosa, un extrait de pétales de souci, un extrait de barbitamao, un extrait de kigelia africana, un extrait d'avoine, les cellules de cacao, la vitamine C, la vitamine E et l'hexylrésorcinol.

12. Utilisation selon l'une quelconque des revendications précédente, **caractérisée en ce que** la composition cosmétique se présente sous forme de gel, d'émulsion, d'émulsion biphasique huile-dans-eau ou eau-dans-huile, d'huile corporelle, de masque, de pommade, d'onguent, de lotion, de solution concentrée, de nanocapsules ou de liposomes.

13. Utilisation selon l'une quelconque des revendications précédentes, **caractérisée en ce que** la composition cosmétique comprend en outre des agents de protection contre les rayons ultraviolets ou des pigments formant écran anti-ultraviolet.

14. Procédé cosmétique non thérapeutique pour améliorer la fermeté et/ou l'élasticité de la peau, consistant à appliquer sur les zones de la peau concernées au moins une composition cosmétique topique contenant une composition huileuse à base d'un extrait lipophile *d'Hemerocallis fulva* et d'un véhicule huileux comprenant au moins une huile végétale.

15. Utilisation non thérapeutique d'une composition cosmétique à application topique comprenant une composition huileuse à base d'un extrait lipophile *d'Hemerocallis fulva* et un véhicule huileux renfermant au moins une huile végétale, pour améliorer la fermeté et/ou l'élasticité de la peau.

## Patentansprüche

1. Verwendung, als aktiven Wirkstoff, einer öligen Zusammensetzung auf der Basis eines lipophilen Extrakts von *Hemerocallis fulva* und eines öligen Vehikels, umfassend mindestens ein Pflanzenöl, für die Zubereitung einer kosmetischen Zusammensetzung zur topischen Anwendung, die bestimmt ist, die Straffheit und/oder die Elastizität der Haut zu verbessern.

2. Verwendung nach Anspruch 1, **dadurch gekennzeichnet, dass** die ölige Zusammensetzung 0,1 bis 10 Ma% in Bezug zur Gesamtmasse der kosmetischen Zusammensetzung darstellt.

3. Verwendung nach Anspruch 1, **dadurch gekennzeichnet, dass** die ölige Zusammensetzung 0,5 bis 4 Ma% in Bezug zur Gesamtmasse der kosmetischen Zusammensetzung darstellt.

4. Verwendung nach einem der vorangehenden Ansprüche, **dadurch gekennzeichnet, dass** der Extrakt teilweise aus den oberirdischen Teilen von *Hemerocallis fulva* gewonnen wird.

5. Verwendung nach einem der vorangehenden Ansprüche, **dadurch gekennzeichnet, dass** der Extrakt durch Extraktion, geschützt vor Luft, gewonnen wird.

6. Verwendung nach einem der vorangehenden Ansprüche, **dadurch gekennzeichnet, dass** das ölige Vehikel mindestens ein natürliches Pflanzenöl umfasst, ausgewählt aus dem Avocadoöl, dem Muskatrosenöl, dem Haselnussöl, dem Rapsöl, dem Macadamiaöl, dem Arganöl, dem Sonnenblumenöl, dem Leindotteröl, dem Borretschöl, dem Süßmandelöl, dem Färberdistelöl, dem Calophyllumöl und ihren Gemischen.

7. Verwendung nach Anspruch 6, **dadurch gekennzeichnet, dass** das natürliche Pflanzenöl aus dem Avocadoöl, dem Muskatrosenöl und ihren Gemischen ausgewählt ist.

8. Verwendung nach Anspruch 7, **dadurch gekennzeichnet, dass** das ölige Vehikel ein Gemisch aus Avocadoöl und Muskatrosenöl umfasst.

9. Verwendung nach Anspruch 8, **dadurch gekennzeichnet, dass** das Massenverhältnis Avocadoöl/Muskatrosenöl innerhalb der öligen Zusammensetzung von 4/1 zu 1/1 schwankt.

10. Verwendung nach einem der vorangehenden Ansprüche, **dadurch gekennzeichnet, dass** der Gehalt an lipophilem Hemerocallis-Extrakt von 5 bis 20 ppm schwankt.

11. Verwendung nach einem der vorangehenden Ansprüche, **dadurch gekennzeichnet, dass** die kosmetische Zusammensetzung ferner einen oder mehrere sekundäre Wirkstoffe enthält, die aus einem Anti-Age-Mittel, dem Acetyltetrapeptid-5, einem Planktonextrakt, einem Macaextrakt, einem Mohnblumenblütenblätterextrakt, einer Kombination von Extrakten von Ochsenzunge, Mohnblume und Passionsblume, einem Mimosensamenextrakt, einem Ringelblumenblütenblätterextrakt, einem Barbitamaoextrakt, einem Leberwurstbaumextrakt, einem Haferextrakt, den Kakaozellen, dem Vitamin C, dem Vitamin E und dem Hexylresorcinol ausgewählt sind.

12. Verwendung nach einem der vorangehenden Ansprüche, **dadurch gekennzeichnet, dass** die kosmetische Zusammensetzung in Form von Gel, Emulsion, Zweiphasen-ÖI-in-Wasser- oder -Wasser-in-ÖI-Emulsion, Körperöl, Maske, Pomade, Salbe, Lotion, Lösungskonzentrat, Nanokapseln oder Liposomen vorliegt.

13. Verwendung nach einem der vorangehenden Ansprüche, **dadurch gekennzeichnet, dass** die kosmetische Zusammensetzung ferner Schutzmittel gegen ultraviolette Strahlen oder Pigmente, die einen Anti-Ultraviolett-Schirm bilden, umfasst.

14. Nicht therapeutisches kosmetisches Verfahren zur Verbesserung der Straffheit und/oder Elastizität der Haut, das darin besteht, auf die betroffenen Hautbereiche mindestens eine topische kosmetische Zusammensetzung aufzutragen, die eine ölige Zusammensetzung auf der Basis eines lipophilen Extrakts von *Hemerocallis fulva* und eines öligen Vehikels, das mindestens ein Pflanzenöl umfasst, enthält.

15. Nicht therapeutische Verwendung einer kosmetischen Zusammensetzung für die topische Anwendung, umfassend eine ölige Zusammensetzung auf der Basis eines lipophilen Extrakts von *Hemerocallis fulva* und ein öliges Vehikel, einschließend mindestens ein Pflanzenöl, zur Verbesserung der Straffheit und/oder der Elastizität der Haut.

## Claims

1. Use, as active ingredient, of an oily composition based on a lipophilic extract of *Hemerocallis fulva* and an oily vehicle comprising at least one vegetable oil, to prepare a cosmetic composition for topical use intended to improve skin firmness and/or elasticity.

2. The use according to claim 1, **characterized in that** the oily composition represents 0.1 to 10 % by weight relative to the total weight of the cosmetic composition.

3. The use according to claim 1, **characterized in that** the oily composition represents 0.5 to 4 % by weight relative to the total weight of the composition.

4. The use according to any of the preceding claims, **characterized in that** said extract is obtained from the above-ground parts of *Hemerocallis fulva.*

5. The use according to any of the preceding claims, **characterized in that** said extract is obtained by airtight extraction.

6. The use according to any of the preceding claims, **characterized in that** the oily vehicle comprises at least one natural vegetable oil selected from among avocado oil, rose hip oil, hazelnut oil, rapeseed oil, macadamia oil, argan oil, sunflower seed oil, camelina oil, borage oil, sweet almond oil, safflower oil, tamanu oil and mixtures thereof.

7. The use according to claim 6, **characterized in that** the natural vegetable oil is selected from among avocado oil, rose hip oil and mixtures thereof.

8. The use according to claim 7, **characterized in that** the oily vehicle comprises a mixture of avocado oil and rose hip oil.

9. The use according to claim 8, **characterized in that** the weight ratio of avocado oil/rose hip oil in the oily composition varies from 4:1 to 1:1.

10. The use according to any of the preceding claims, **characterized in that** the content of lipophilic *Hemerocallis* extract varies from 5 to 20 ppm.

11. The use according to any of the preceding claims, **characterized in that** the cosmetic composition also contains one or more secondary active ingredients selected from among an anti-ageing agent, acetyl-tetrapeptide-5, a plankton extract, maca extract, poppy petal extract, a combination of extracts of Anchusa, poppy and Passiflora, extract of mimosa seeds, marigold petal extract, Barbitamao extract, extract of Kigelia Africana, oat extract, cocoa cells, vitamin C, vitamin E and hexylresorcinol.

12. The use according to any of the preceding claims, **characterized in that** the cosmetic composition is in the form of a gel, emulsion, biphasic oil-in-water or water-in-oil emulsion, body oil, mask, ointment, unguent, lotion, concentrated solution, nanocapsules or liposomes.

13. The use according to any of the preceding claims, **characterized in that** the cosmetic composition further comprises protective agents against ultraviolet radiation or pigments forming an antiultraviolet screen.

14. Non-therapeutic cosmetic method to improve skin firmness and/or elasticity whereby at least one topical cosmetic composition is applied to the skin regions concerned, containing an oily composition based on a lipophilic extract of *Hemerocallis fulva* and an oily vehicle comprising at least one vegetable oil.

15. Non-therapeutic use of a cosmetic composition for topical application comprising an oily composition based on a lipophilic extract of *Hemerocallis fulva* and an oily vehicle containing at least one vegetable oil, to improve skin firmness and/or elasticity.
